# EUROPEAN PATENT APPLICATION

(11) **EP 3 260 548 A1**
(43) Date of publication of application: **27.12.2017**
(21) Application number: 16175473.4
(22) Date of filing: 21.06.2016
(51) Int. Cl.: C12P 21/02, C07K 14/62

(54) **ENZYMATIC COUPLING OF (OLIGO)PEPTIDES TO THE B-CHAIN OF AN INSULIN RECEPTOR LIGAND**

(71) Applicant: Enzypep B.V., 6167 RD Geleen (NL)
(72) Inventor: QUAEDFLIEG, Peter, Jan, Leonard, Mario, 6167 RD Geleen (NL); NUIJENS, Timo, 6167 RD Geleen (NL); TOPLAK, Ana, 6167 RD Geleen (NL)
(74) Representative: V.O.

(57) **Abstract**

The present invention relates to a method for enzymatically coupling an (oligo)peptide C-terminal ester or thioester to an insulin or other insulin receptor ligand having a first peptide chain comprising an N-terminal glycine residue (also known as an A-chain) and a second peptide chain having an N-terminal phenylalanine residue (also known as a B-chain), wherein the C-terminal ester or thioester is coupled at a higher rate to the N-terminal phenylalanine residue of the second peptide chain than to the N-terminal glycine residue of the first peptide chain
which coupling is carried out in an aqueous fluid wherein the N-terminal glycine residue is at least substantially shielded by a remaining part of the insulin or other insulin receptor ligand, and
which coupling is catalysed by a subtilisin BPN' variant or a homologue thereof, which comprises the following mutations compared to subtilisin BPN' represented by SEQUENCE ID NO: 2 or a homologue sequence thereof:
- a deletion of the amino acids corresponding to positions 75-83;
- a mutation at the amino acid position corresponding to S221, the mutation being S221C or S221selenocysteine;
- a mutation at the amino acid position corresponding to P225 selected from the group of P225N, P225D, P225S, P225C, P225G, P225A, P225T, P225V, P225I an P225L;
- a mutation at the amino acid position corresponding to M222 selected from the group M222P and M222G;
- a mutation at the amino acid position corresponding to Y217 selected from the group of Y217F, Y217H, Y217G, Y217L, Y217A, Y217N, Y217E,, Y217R , Y217K and Y217P.

## Description

The invention relates to a method for enzymatically coupling an (oligo)peptide C-terminal ester or thioester to an insulin or other insulin receptor ligand.

Insulins play a variety of roles in controlling processes such as metabolism, growth and differentiation, and reproduction. On a cellular level they affect cell cycle, apoptosis, cell migration, and differentiation. Insulin acts on insulin receptors. These are transmembrane cell receptors that play a key role in the regulation of glucose homeostasis. In particular, insulin thereby promotes the absorption of glucose from the blood into fat, liver and skeletal muscle cells. Further actions include cellular effects, such as a positive effect on glycogen synthesis in the liver. Lowered levels of insulin cause liver cells to convert glycogen to glucose and excrete it into the blood. This is the clinical action of insulin, which is directly useful in reducing high blood glucose levels. A disturbance in the regulation of the glucose homeostasis may result in a variety of clinical manifestations, such as diabetes or cancer.

Insulin is thus a key drug in the treatment of diabetes. Porcine insulin has been used to treat diabetes type I before recombinant DNA technologies for the production of human insulin became available on a large scale.

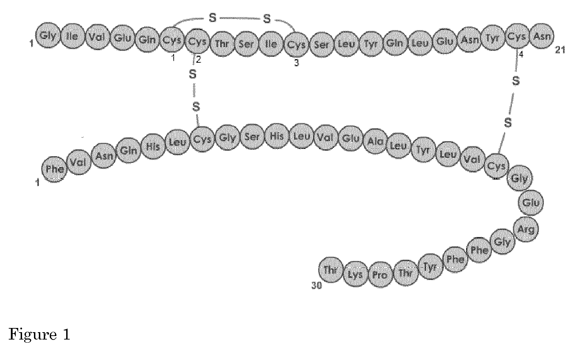

The active form of insulin is composed of two chains (A and B) linked by two disulphide bonds; the arrangement of four cysteines is conserved in the "A" chain: Cys1 is linked by a disulphide bond to Cys3, Cys2 and Cys4 are linked by interchain disulphide bonds to cysteines in the "B" chain; see Figure 1. This alignment contains both chains, plus an intervening linker region, arranged as found in the propeptide form. Propeptides are cleaved to yield two separate chains linked covalently by the two disulphide bonds.

Within vertebrates insulin is highly conserved. The human insulin protein is composed of 51 amino acids, 21 in the A-chain (amino acid sequence H-Gly-Ile-Val-Glu-Gln-Cys-Cys-Thr-Ser-Ile-Cys-Ser-Leu-Tyr-Gln-Leu-Glu-Asn-Tyr-Cys-Asn-OH _and 30 in the B-chain (amino acid sequence: (amino acid sequence H-Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Tyr-Thr-Pro-Lys-Thr-OH). It has a molecular mass of 5808 Da. Porcine insulin has only one mutation compared to human insulin and bovine insulin has three mutations.

Further, altered forms of insulin have been developed, having a different absorption, distribution, metabolism or excretion (ADME) characteristic. Like natural insulin the altered forms participate in the activation of insulin receptors. Accordingly, insulins of various species and altered forms thereof, are referred to in the art as insulin receptor ligands, or - more colloquially - as insulin analogues.

It is in particular desirable to provide insulin receptor ligands for specific aspects of glycemic control in terms of fast action (prandial insulins) and long action (basal insulins).

Examples of fast acting (quickly absorbed) insulin receptor ligands are Humalog (insulin lispro™), aspart (NovoRapid™) and insulin glulisine (Apidra™).

In Lispro the penultimate lysine and proline residues on the C-terminal end of the B-chain are reversed.

In aspart, the proline residue at position 28 of the B-chain is substituted with an aspartic acid residue.

Insulin glulisine differs from human insulin in that the amino acid asparagine at position 3 of the B-chain is replaced by lysine and the lysine in position 29 of the B-chain is replaced by glutamic acid.

In addition to or alternately to providing an insulin receptor ligand with a modified amino acid sequence, it is possible to alter an ADME characteristic by modifying a functional group (in particular an amino group, carboxylic acid group or hydroxyl group) on a side chain of an amino acid of one or both of the A-chain and B-chain.

For example, insulin detemir (Levemir™) is an insulin-like protein in which a fatty acid (myristic acid) is bound to the lysine amino acid at position B29. It is quickly absorbed after which it binds to albumin in the blood through its fatty acid at position B29. It then slowly dissociates from this complex.

Insulin degludec is a modified insulin that has one single amino acid (Thr30) deleted in comparison to human insulin, and is conjugated to hexadecanedioic acid via a gamma-L-glutamyl spacer at the amino acid lysine at position B29.

Glargine insulin (Lantus™) is a long-lasting insulin receptor ligand. Two arginines are added to the C-terminus of the B-chain, and they shift the isoelectric point from 5.4 to 6.7, making glargine more soluble at a slightly acidic pH and less soluble at a physiological pH. Asparagine at position 21 in the A-chain is replaced by glycine to avoid deamination. It is formulated with zinc.

Further insulin analogous have been described in, e.g., EP-A 2 6784 085, EP-A 425 482 and EP-A 419 504.

It is also possible to couple another (oligo)peptide to the N-terminal amino acid of the A-chain (glycine) or to both the N-terminal amino acid of the A-chain (glycine) and the B-chain (phenyl alanine). Such molecule may be used to alter an ADME characteristic, e.g. increase solubility in blood plasma or increase half-life of the insulin receptor ligand. Another reason to couple another (oligo)peptide to the insulin receptor ligand is to provide it with another pharmaceutically active agent. It would be desirable to selectively couple a compound to the N-terminal end of the B-chain because the A-chain is usually much more important for the biological activity of the insulin receptor ligand, and thus a coupling to the B-chain may be used to alter another property of the ligand without substantially altering such biological activity, in particular binding to the insulin receptor.

However, it is a challenge to couple an (oligo)peptide to the B-chain in general. Chemical methods are not suitable to accomplish this in a selective manner, since the insulin receptor ligand is usually produced fermentatively. Known enzymatic coupling methodology has not shown to be selective towards the N-terminal end of the B-chain either. If any coupling is accomplished at all, it is the N-terminal glycine of the A-chain that is preferentially used as a coupling site.

In WO 2016/056913, two examples are shown of effective enzymatic coupling of a pentapeptide to human insulin. Dependent on the used enzyme, either only coupling takes place at the N-terminal glycine of the A-chain, or coupling takes place a both the N-terminal end of the A-chain and of the B-chain, with a higher selectivity towards the N-terminal end of the A-chain.

It would be desirable to provide an alternative methodology to couple an (oligo)peptide to the N-terminal phenylalanine of the B-chain of an insulin receptor ligand, in particular methodology that allows improved selectivity towards coupling to the N-terminal phenylalanine of the B-chain relative to coupling to the N-terminal glycine of the A-chain, more in particular at a satisfactory yield within a reasonable reaction time.

It has now been found possible to enzymatically couple an (oligo)peptide selectively to the N-terminal phenylalanine of the B-chain of an insulin receptor ligand taking special precautions with respect to the reaction medium wherein the (oligo)peptide is coupled to the insulin receptor ligand in a reaction catalysed by a subtilisin BPN' variant or homologue thereof.

Accordingly, the invention relates to method for enzymatically coupling an (oligo)peptide C-terminal ester or thioester to an insulin or other insulin receptor ligand having a first peptide chain comprising an N-terminal glycine residue (also known as an A-chain) and a second peptide chain having an N-terminal phenylalanine residue (also known as a B-chain), wherein the C-terminal ester or thioester is coupled at a higher rate to the N-terminal phenylalanine residue of the second peptide chain than to the N-terminal glycine residue of the first peptide chain,
which coupling is carried out in an aqueous fluid,
which coupling is catalysed by a subtilisin BPN' variant or a homologue thereof. The subtilisin BPN' variant or a homologue thereof comprises the following mutations compared to subtilisin BPN' represented by SEQUENCE ID NO: 2 or a homologue sequence thereof:
- a deletion of the amino acids corresponding to positions 75-83;
- a mutation at the amino acid position corresponding to S221, the mutation being S221C or S221selenocysteine;
- a mutation at the amino acid position corresponding to P225 selected from the group of P225N, P225D, P225S, P225C, P225G, P225A, P225T, P225V, P225I an P225L. Further, the subtilisin BPN' variant or a homologue thereof typically comprises
- a mutation at the amino acid position corresponding to M222 selected from the group M222P and M222G;
- a mutation at the amino acid position corresponding to Y217 selected from the group of Y217F, Y217H, Y217G, Y217L, Y217A, Y217N, Y217E,, Y217R , Y217K and Y217P
wherein the amino acid positions are defined according to the sequence of subtilisin BPN' represented by SEQUENCE ID NO: 2.

Figure 1 schematically shows the amino acid sequence of the A-chain and the B-chain of human insulin plus the disulphide bridges between the A-chain and the B-chain, as well as a disulphide bond between to cysteine residues of the A-chain.

In accordance with the invention, in the aqueous fluid wherein the coupling takes place, the N-terminal glycine residue of the A-chain is at least substantially shielded by a remaining part of the insulin or other insulin receptor ligand.. I.e. it is at least substantially protected by a remaining part of the insulin receptor ligand from participating in the enzymatic coupling reaction. The shielding effect of the remaining part of the insulin receptor ligand means that the C-terminal ester or thioester is coupled at a higher rate to the N-terminal phenylalanine residue of the B-chain than to the N-terminal glycine residue of the A-chain, in particular at an at least two times higher rate. Preferably, the (molar) ratio of the coupling towards said N-terminal phenylalanine to said N-terminal glycine is at least 3:1, more preferably at least 6:1, most preferably at least 10:1. In principle one may carry out the method to obtain a product that is essentially free of product wherein (oligo)peptide ester is coupled to the A-chain. In practice, it may be desired to allow some coupling to the A-chain, e.g. in embodiments where this may contribute to a higher overall conversion rate. Thus, in practice, one may carry out a coupling method of the invention, wherein the (molar) ratio of the coupling towards said N-terminal phenylalanine to said N-terminal glycine is about 100:1 or less.

It has been found possible, to obtain a coupling product of (oligo)peptide ester or thioester and insulin receptor ligand comprising a conjugate having a single (oligo)peptide coupled to the insulin receptor ligand, which single (oligo)peptide is coupled the N-terminal phenylalanine of the B-chain, in a relative amount of more than 50 mol %, in particular in a relative amount of at least 80 mol %, more in particular of at least 90 mol %, based on total coupling product (i.e. the total of conjugates of the (oligo)peptides coupled to the insulin receptor ligand), without needing any separation or other recovery step. The relative amount of the conjugate having a single (oligo)peptide coupled to the insulin receptor ligand, which single (oligo)peptide is coupled the N-terminal phenylalanine of the B-chain is up to 100 %, based on total coupling product, e.g. 98 mol % or less, or 96 mol % or less. In particular, it has been found possible to obtain a coupling product that is essentially free of conjugate wherein both an (oligo)peptide has been coupled to the N-terminal glycine of the A-chain and an (oligo)peptide has been coupled to the N-terminal phenylalanine of the B-chain of the insulin receptor ligand.

Thus, the present invention provides a method for synthesising a conjugate of an (oligo)peptide and an insulin receptor ligand, said conjugate having a single (oligo)peptide coupled to the insulin receptor ligand, which single (oligo)peptide is coupled the N-terminal phenylalanine of the B-chain of the insulin receptor ligand, which synthesis is carried out in an aqueous fluid and catalysed by the subtilisin BPN' variant or a homologue thereof comprising said mutations compared to subtilisin BPN' represented by SEQUENCE ID NO: 2 or a homologue sequence, e.g., as illustrated by SEQUENCE ID NO: 3 or SEQUENCE ID NO: 4.

It is surprising that it is possible to enzymatically couple an (oligo)peptide to the N-terminal phenylalanine of the B-chain of an insulin receptor ligand with improved selectivity, without needing to chemically protect the glycine, and that this is possible with a relatively high yield in a reasonably short time. It is e.g. possible to accomplish this in a fully aqueous fluid, which is generally unfavourable to the synthesis over hydrolysis ratio in condensation reactions.

For the purpose of this invention, with "synthesis over hydrolysis ratio" (S/H ratio) is meant the amount of enzymatically synthesised (oligo)peptide product divided by the amount of (oligo)peptide C-terminal ester or thioester of which the ester or thioester group has been hydrolysed.

The value of the S/H ratio of an enzyme of the invention depends on various factors, for instance the nature of the substrates (the amino acid sequences of the (oligo)peptide C-terminal ester or thioester and of the (oligo)peptide nucleophile) and reaction conditions (e.g. temperature, pH, concentration of the peptide fragments, enzyme concentration).

Further, using a method of the invention, the (oligo)peptide-insulin receptor ligand condensation product is very easy to purify from the reaction mixture because only little hydrolytic by-products are formed and because little or no (oligo)peptide is coupled to the A-chain.

The term "or" as used herein is defined as "and/or" unless it is specified otherwise or it follows from the context that it means 'either ....or...' .

The term "a" or "an" as used herein is defined as "at least one" unless it is specified otherwise or it follows from the context that it should refer to the singular only.

When referring to a noun (e.g. a compound, an additive, etc.) in the singular, the plural is meant to be included, unless it follows from the context that it should refer to the singular only.

For the purpose of this invention, with "peptides" is meant any chain composed of two or more amino acids. Thus, peptides are generally amides at least conceptually composed of two or more amino carboxylic acid molecules (i.e. amino acids) by formation of a covalent bond from the carbonyl carbon of one to the nitrogen atom of another with formal loss of water. The term is usually applied to structures formed from alpha-amino acids. A peptide may be linear, branched or cyclic. A peptide can have a single chain composed of two or more amino acids or a peptide can have a plurality of chains. In the case a peptide is composed of two or more chains, each chain generally is composed of three or more amino acid molecules. The amino acid sequence of a peptide is referred to as the primary structure.

In an embodiment, the (oligo)peptide is essentially free of a secondary structure and essentially free of a tertiary structure.

In a further embodiment, the (oligo)peptide has a secondary structure. Secondary structures are generally highly regular local sub-structures, such as alpha-helices and beta-sheets (or beta-strands), by interactions between the individual amino acids and the peptide backbone.

In an embodiment, the (oligo)peptide (or plurality of peptides) has a tertiary structure. Tertiary structures are generally formed by multiple interactions, among others hydrogen bonding, hydrophobic interactions, van der Waals interactions, ionic interactions and disulphide bonds. The secondary structure can also contribute to the tertiary structure. The tertiary structure provides a three-dimensional shape (which is essentially fixed in a stable environment, such as in the absence of a change in temperature and in the absence of a change in the medium wherein the peptide is present, etc.). As the skilled person knows, the tertiary structure is different from a random coil peptide chain lacking any fixed three-dimensional structure. Proteins, like insulin, are (oligo)peptides having a tertiary structure.

In an embodiment, the (oligo)peptide to be coupled to the insulin receptor ligand is a protein for target delivery of the insulin receptor ligand to a specific site, e.g. to organ tissue. Well known examples of proteins, suitable for such purpose, are immunoglobulins or parts thereof, such as an antigen-binding fragment (Fab) of an immunoglobulin. In an embodiment, the (oligo)peptide is a protein suitable to increase the half-life of the insulin receptor ligand in a living organism, in particular the blood plasma half-life. Albumins are examples of proteins that can be coupled to other peptides to increase the half-life.

Disulphide bonds (disulphide bridges) are typically bonds between two cysteine units (formed by oxidation). Thus, two amino acids in a same peptide chain (amino acid sequence) can be covalently bound, also if they are not adjacent amino acids in the amino acid sequence. Also, a disulphide bond between a first cysteine of a first peptide chain and a second cysteine of a second peptide chain, which may have the same or a different amino acid sequence, can be formed to form a peptide. Such peptide comprises more than one peptide chain. An example of a peptide composed of more than one peptide chain, wherein the different chains are bound via a disulphide bond is insulin.

In an embodiment, the (oligo)peptide to be coupled to the insulin receptor ligand essentially consists of amino acid units.

In a further embodiment, the (oligo)peptide essentially consists of amino acid units and protective groups.

In an embodiment, the (oligo)peptide is a conjugate of a peptide chain of two or more amino acids and another residue, such as a carobohydrate or a lipid. These peptides are called glycopeptides and lipopeptides respectively.

In a further embodiment, the (oligo)peptide to be coupled to the insulin receptor ligand is an (oligo)peptide modified with a synthetic hydrophilic polymer, such as a polyalkylene glycol. Particularly preferred polyalkylene glycols are polyethylene glycols.

In a further embodiment, the (oligo)peptide to be coupled is a conjugate of an (oligo)peptide and a fatty acid, e.g. a C8-C24 saturated or unsaturated fatty acid.

In a further embodiment, the (oligo)peptide to be coupled is a conjugate of an (oligo)peptide and a polysialic acid.

In a further embodiment, the (oligo)peptide is a conjugate of an (oligo)peptide and an imaging agent, such as a fluorescent, phosphorescent, chromogenic or radioactive group. The peptide conjugate may also contain a chelation agent or toxin.

Typically, a peptide - which term includes oligopeptides, proteins and peptide conjugates - comprises up to about 35 000 amino acid units, in particular 3-20 000 amino acid units, more in particular 4-5 000 amino acid units, preferably 5-1000 amino acid units. In a specifically preferred embodiment the peptide comprises 500 amino acid units or less, in particular 200 or less, more in particular 100 or less. In a specifically preferred embodiment, the peptide comprises at least 10 amino acid units, more specifically at least 15 amino acids, at least 25 amino acids or at least 40 amino acids.

With "oligopeptides" is meant within the context of the invention, a peptide composed of 2-200 amino acid units, in particular composed of 5-100 amino acid units, more in particular composed of 10-50 amino acid units.

The term "(oligo)peptide" is used herein as a short-hand for the phrase "peptides, in particular oligopeptides".

For the purpose of this invention, with "peptide bond" is meant the amide bond between (i) either the alpha-amino terminus of one alpha-amino acid or the beta-amino acid terminus of one beta-amino acid and (ii) either the alpha-carboxyl terminus of one other alpha-amino acid or the beta-carboxyl terminus of one other beta-amino acid. Preferably, the peptide bond is between the alpha-amino terminus of one amino acid and the alpha-carboxyl terminus of another amino acid.

For the purpose of this invention, with "condensation" is meant the formation of a new peptide bond between the C-terminal carboxylic function of an (oligo)peptide with the N-terminal amine function of another (oligo)peptide or of the same (oligo)peptide.

In the context of this application, the term "about" means in particular a deviation of 10 % or less from the given value, more in particular 5 % or less, even more in particular 3 % or less.

The term "essential(ly)" or "(at least) substantial(ly)" is generally used herein to indicate that it has the general character or function of that which is specified. When referring to a quantifiable feature, this term is in particular used to indicate that it is at least 75 %, more in particular more than 90 %, even more in particular more than 98 % of the maximum that feature. The term "essentially free" is generally used herein to indicate that a substance is not present (below the detection limit achievable with analytical methodology as available on the effective filing date) or present in such a low amount that it does not significantly affect the property of the product that is essentially free of said substance. In practice, in quantitative terms, a product is usually considered essentially free of a substance, if the content of the substance is 0 - 0.1 wt.%, in particular 0 - 0.01 wt.%, more in particular 0 - 0.005 wt.%, based on total weight of the product in which it is present.

In the context of the invention with "amino acid side-chain" is meant any proteinogenic or non-proteinogenic amino acid side-chain.

The term "mutated" or "mutation" as used herein regarding proteins or polypeptides - in particular enzymes - means that at least one amino acid in the wild-type or naturally occurring protein or polypeptide sequence has been replaced with a different amino acid, inserted into, appended to, or deleted from the sequence via mutagenesis of nucleic acids encoding these amino acids. Mutagenesis is a well-known method in the art, and includes, for example, site-directed mutagenesis by means of PCR or via oligonucleotide-mediated mutagenesis as described in Sambrook et al., Molecular Cloning-A Laboratory Manual, 2nd ed., Vol. 1-3 (1989). The term "mutated" or "mutation" as used herein regarding genes means that at least one nucleotide in the nucleic acid sequence of that gene or a regulatory sequence thereof, has been replaced with a different nucleotide, has been inserted into, has been appended to, or has been deleted from the sequence via mutagenesis, resulting in the transcription of a protein sequence with a qualitatively of quantitatively altered function or resulting in the knock-out of that gene.

In the present specification, a shorthand for denoting amino acid substitutions employs the single letter amino acid code of the amino acid that is substituted, followed by the number designating where in the protein amino acid sequence the substitution is made. This number is the amino acid position of the wild-type amino acid sequence (generally subtilisin BPN' unless specified otherwise). Thus for the mutated amino acid sequence it is the amino acid position corresponding to the position with that number in the wild type enzyme. Due to one or more other mutations at a lower position (additions, insertions, deletions, etc.) the actual position does not need to be the same. The skilled person will be able to determine the corresponding positions using a generally known alignment technique, such as NEEDLE. The number is followed by the single letter code of the amino acid that replaces the wild-type amino acid therein. For example, G166S denotes the substitution of glycine at the position corresponding to position 166 to serine. X is used to indicate any other proteinogenic amino acid than the amino acid to be substituted. For example, G166X denotes the substitution of glycine 166 to any other proteinogenic amino acid.

When referring to a compound of which stereoisomers exist, the compound may be any of such stereoisomers or a mixture thereof. Thus, when referred to, e.g., an amino acid of which enantiomers exist, the amino acid may be the L-enantiomer, the D-enantiomer or a mixture thereof. In case a natural stereoisomer exists, the compound is preferably a natural stereoisomer.

The term 'pH' is used herein for the apparent pH, i.e. the pH as measured with a standard, calibrated pH electrode.

When an enzyme is mentioned with reference to an enzyme class (EC) between brackets, the enzyme class is a class wherein the enzyme is classified or may be classified, on the basis of the Enzyme Nomenclature provided by the Nomenclature Committee of the International Union of Biochemistry and Molecular Biology (NC-IUBMB), which nomenclature may be found at http://www.chem.qmul.ac.uk/iubmb/enzyme/. Other suitable enzymes that have not (yet) been classified in a specified class but may be classified as such, are meant to be included.

Homologues typically have an intended function in common with the peptide (e.g. enzyme or insulin receptor ligand) of which it is a homologue, such as being capable of catalyzing the same reaction or being a ligand to the same receptor.

Amino acid or nucleotide sequences are said to be homologous when exhibiting a certain level of similarity. Two sequences being homologous indicate a common evolutionary origin. Whether two homologous sequences are closely related or more distantly related is indicated by "percent identity" or "percent similarity", which is high or low respectively.

The terms "homology", "percent homology", "percent identity" or "percent similarity" are used interchangeably herein. For the purpose of this invention, it is defined here that in order to determine the percent identity of two amino acid sequences, the complete sequences are aligned for optimal comparison purposes. In order to optimize the alignment between the two sequences gaps may be introduced in any of the two sequences that are compared. Such alignment is carried out over the full length of the sequences being compared. Alternatively, the alignment may be carried out over a shorter length, for example over about 20, about 50, about 100 or more nucleic acids or amino acids. The percentage identity is the percentage of identical matches between the two sequences over the reported aligned region.

A comparison of sequences and determination of percent identity between two sequences can be accomplished using a mathematical algorithm. The skilled person will be aware of the fact that several different computer programs are available to align two sequences and determine the homology between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley). The percent identity between two amino acid sequences can be determined using the Needleman and Wunsch algorithm for the alignment of two sequences. (Needleman, S. B. and Wunsch, C. D. (1970) J. Mol. Biol. 48, 443-453). The Needleman-Wunsch algorithm has been implemented in the computer program NEEDLE. For the purpose of this invention the NEEDLE program from the EMBOSS package was used (version 2.8.0 or higher, EMBOSS: The European Molecular Biology Open Software Suite (2000) Rice,P. Longden,I. and Bleasby,A. Trends in Genetics 16, (6) pp276-277, http://emboss.bioinformatics.nl/). For protein sequences, EBLOSUM62 is used for the substitution matrix. Other matrices can be specified. The optional parameters used for alignment of amino acid sequences are a gap-open penalty of 10 and a gap extension penalty of 0.5. The skilled person will appreciate that all these different parameters will yield slightly different results but that the overall percentage identity of two sequences is not significantly altered when using different algorithms.

The homology or identity between the two aligned sequences is calculated as follows: the number of corresponding positions in the alignment showing an identical amino acid in both sequences divided by the total length of the alignment after subtraction of the total number of gaps in the alignment. The identity defined as herein can be obtained from NEEDLE by using the NOBRIEF option and is labelled in the output of the program as "longest-identity". For purposes of the invention the level of identity (homology) between two sequences is calculated according to the definition of "longest-identity" as can be carried out by using the program NEEDLE.

The polypeptide sequences can further be used as a "query sequence" to perform a search against sequence databases, for example to identify other family members or related sequences. Such searches can be performed using the BLAST programs. Software for performing BLAST analyses is publicly available through the National Center for Biotechnology Information (http://www.ncbi.nlm.nih.gov). BLASTP is used for amino acid sequences. The BLAST program uses as defaults:
- Cost to open gap: default = 11 for proteins
- Cost to extend gap: default = 1 for proteins
- Expect value: default = 10
- Wordsize: default = 28 for megablast/ 3 for proteins

Furthermore the degree of local identity (homology) between the amino acid sequence query and the retrieved homologous sequences is determined by the BLAST program. However only those sequence segments are compared that give a match above a certain threshold. Accordingly the program calculates the identity only for these matching segments. Therefore the identity calculated in this way is referred to as local identity.

The term "homologue" is used herein in particular for peptides (enzymes, proteins) having a sequence identity of at least 50 %, preferably at least 60 %, more preferably at least 70 %, at least 80 %, at least 90 %, at least 95 %, at least 96 %, at least 97 %, at least 98 % or at least 99 % with the peptide with which the homologue peptide is compared. Evidently, the sequence identity will be less than 100 %. The percentage of sequence identity will depend on the number of mutations and the length of the peptide with which the homologue is prepared. In particular, for a subtilisin BPN' variant, the number of mutations will typically be at least 13, of which at least nine mutations are deletions and at least four mutations are replacements for another amino acid. In 'longest identity' alignment the deletions are not taken into account. Preferably, the sequence identity of an enzyme of the invention compared to SEQUENCE ID NO 2, is 98 % or less, more preferably 96 % or less, in particular 94 % or less, more in particular 92 % or less, or 90 % or less.

For the purpose of clarity and a concise description features are described herein as part of the same or separate embodiments, however, it will be appreciated that the scope of the invention may include embodiments having combinations of all or some of the features described. Terms used herein that are not specifically defined herein are as defined in WO 2016/056913, or - if not defined therein - used in accordance with common general knowledge.

The term 'C-terminal protection' is used herein to indicate that a C-terminal carboxylic group of an (oligo)peptide is provided with a protective group, generally substantially protecting the carboxylic group from being coupled to an N-terminal amine group of another (oligo)peptide or of the same (oligo)peptide molecule. The C-terminal protective group may be a t-alkyl ester group for instance a t-butyl ester group, which is a commonly used protective group. The C-terminal protective group may also be a C-terminal carboxy-amide. A primary carboxy-amide is a commonly used protective group.

The term '*N*-terminal protection' is used herein to indicate that an *N-*terminal amine group of an (oligo)peptide is provided with a protective group, generally at least substantially protecting the N-terminal amine group from being coupled to a C-terminal carboxylic group of another (oligo)peptide or of the same (oligo)peptide molecule.

The (oligo)peptide C-terminal ester or thioester typically is an activated (thio)ester, i.e. it contains a carboxy ester or carboxy thioester group that can take part in the enzymatic coupling reaction. In principle, any (substituted or unsubstituted) alkyl or (substituted or unsubstituted) aryl (thio)ester can be used. Typical examples of (thio)esters which can take part in the enzymatic coupling reaction are methyl-, ethyl, propyl-, isopropyl-, phenyl-, benzyl-, 2,2,2-trichloroethyl-, 2,2,2-trifluoroethyl-, cyanomethyl- and carboxyamidomethyl-(thio)esters.

Particularly good results have been obtained with carboxyamidomethyl-type esters represented by the formula peptide-(C=O)-O-CX₁X₂-C(=O)N-R₁R₂. Herein, each X₁ and X₂ independently represents a hydrogen atom or an alkyl group. Good results have been achieved when both X₁ and X₂ are a hydrogen atom (peptide-(C=O)-O-CH₂-C(=O)N-R₁R₂). Herein R₁ represents a hydrogen atom or an alkyl group and R₂ represents a hydrogen atom or an alkyl group or an amino acid or a peptide residue with a C-terminal carboxyamide or carboxylic acid functionality, optionally protected on the side-chain functionality of the amino acid or on one or more of the side-chain functionalities of the amino acids. Herein, each alkyl group may independently represent a (substituted or unsubstituted) C1-C7 alkyl group, preferably a (substituted or unsubstituted) linear C1-C6 alkyl group, more preferably a (substituted or unsubstituted) linear C1-C3 alkyl group, and most preferably a methyl group. Good results have in particular been achieved in a method of the invention wherein both R₁ and R₂ represent a hydrogen atom or wherein R₁ represents a hydrogen atom and R₂ represents an amino acid or peptide residue with a C-terminal carboxyamide or carboxylic acid functionality, optionally protected on the side-chain functionality of the amino acid or on one or more of the side-chain functionalities of the amino acids. Particularly good results have been achieved when using the Cam-ester, when X₁, X₂, R₁ and R₂ are a hydrogen atom.

The (oligo)peptide C-terminal (thio)ester can be N-terminally unprotected or N-terminally protected. In an embodiment, one or more side-chain functionalities (in particular carboxyl groups, amine groups), e.g. all side-chain functionalities, are provided with a protecting group; in another embodiment all the side-chain functionalities are unprotected. In a preferred embodiment, only the side-chain functionalities of the amino acids at the P4 and P1 position of the (oligo)peptide C-terminal (thio)ester (in particular hydroxy groups, carboxyl groups or amine groups) are provided with a protecting group. Suitable protecting groups are known to the person skilled in the art. Carboxylic acid groups can for instance be protected with a cyclohexyl, benzyl or allyl group; amine functionalities can for instance be protected with an allyloxycarbonyl group or a trifluoroacetyl group.

The activated C-terminal (thio)ester group of the (oligo)peptide C-terminal (thio)ester can be synthesized using solid phase synthesis in high yield and purity without racemization. An additional advantage of the use of (thio)esters wherein R₁ represents a hydrogen atom and R₂ represents an amino acid or peptide residue with a C-terminal carboxylic acid functionality, optionally protected on the side-chain functionality of the amino acid or on one or more of the side-chain functionalities of the amino acids is, that their activated C-terminal ester or thioester group can be synthesized using the cheap and industrially available 2-chlorotritylchloride resin.

The activated C-terminal (thio)ester group of the (oligo)peptide C-terminal (thio)ester can also be synthesized by fermentation using a microorganism. A reliable method to obtain (oligo)peptide (thio)esters using fermentation is via so-called intein expression (see for instance E.K. Lee, Journal of Chemical Technology and Biotechnology, 2010, 9, 11-18). Different intein expression systems kits are commercially available (for instance the IMPACT™ kit). Other methods for the fermentative production of (oligo)peptide (thio)esters are known in the art.

The C-terminal amino acid of the (oligo)peptide C-terminal (thio)ester and the other amino acids of the (oligo)peptide C-terminal (thio)ester may in principle be any amino acid, proteinogenic or non-proteinogenic. If the amino acid sequence of the C-terminal part of the (oligo)peptide C-terminal (thio)ester is poorly recognized by or inaccessible to the coupling enzyme due to the amino acid preference of the coupling enzyme and/or due to the secondary or tertiary structure of the (oligo)peptide, the primary structure (amino acid sequence) may be elongated at the C-terminus. Essentially the C-terminus of the (oligo)peptide C-terminal (thio)ester is elongated with a number of amino acids to ensure good recognition by the enzyme and accessibility into the enzyme for the enzymatic coupling reaction. The skilled person will know how to elongate the (oligo)peptide C-terminal (thio)ester on the basis of the information disclosed herein and common general knowledge. Usually the number of amino acids for elongation is in the range of 1-10, although in principle it can be higher. Good results have been obtained by elongation of the (oligo)peptide C-terminal (thio)ester with 4 amino acid residues, e.g. -Phe-Ser-Lys-Leu-(thio)ester.

In particular the (optionally N-terminal protected) (oligo)peptide C-terminal (thio)ester may be represented by a compound of Formula I.

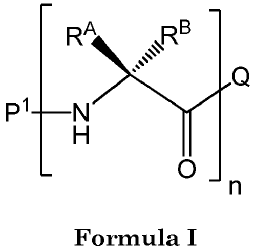

Herein Q represents an OR or SR moiety. R may represent a (substituted or unsubstituted) alkyl or a (substituted or unsubstituted) aryl group.

Herein P¹ stands for a hydrogen or an N-terminal protecting group. Suitable N-terminal protecting groups are those N-protecting groups which can be used for the synthesis of (oligo)peptides. Such groups are known to the person skilled in the art. Examples of suitable N-protecting groups include carbamate or acyl type protecting groups, for instance 'Cbz' (benzyloxycarbonyl), 'Boc' (tert-butyloxycarbonyl), 'For' (formyl), 'Fmoc' (9-fluorenylmethoxycarbonyl), 'PhAc' (phenacetyl) and 'Ac' (acetyl). The groups For, PhAc and Ac may be introduced and cleaved enzymatically using the enzymes Peptide Deformylase, PenG acylase or Acylase, respectively. Chemical cleavage methods are generally known in the art.

Herein, n is an integer of at least 2. n May in particular be at least 3, at least 4, at least 5, at least 6, at least 7 at least 8, at least 9 or at least 10. n May in particular be 100 or less, 75 or less, 50 or less, 25 or less, 20 or less 15 or less, e.g. 10 or less.

Herein, each R^{A} and each R^{B} independently represent a hydrogen atom or an organic moiety, preferably an amino acid side-chain. Thus, it is not required that R^{A} is the same in all n amino acid units. Similarly, it is not required that R^{B} is the same in all n amino acid units. Optionally, one or more of the side-chain functionalities may contain a protecting group.

The (oligo)peptide C-terminal (thio)ester can in principle be widely chosen, dependent on the desired effect. As already indicated above, it can be an oligopeptide, a protein or a conjugate of an (oligo)peptide and another molecule.

The coupling is a condensation reaction wherein the insulin receptor ligand acts as the nucleophile. In an embodiment, the nucleophile is C-terminal protected. In another embodiment it is not C-terminal protected.

In principle, the method can be applied to any insulin. Preferably, the insulin is human insulin. Another particularly suitable insulin is porcine insulin.

As other insulin receptor ligand in principle any insulin receptor ligand may be used having, in active form, insulin receptor activity, with the proviso that the insulin receptor ligand comprising a A-chain having an N-terminal glycine and a B- chain having an N-terminal phenylalanine. Further the insulin receptor ligand generally contains disulphide bonds between A-chain and B-chain, typically at a position corresponding to Cys(A7) and Cys(B7) of human insulin, and between Cys(A20) and Cys(B19) of human insulin; herein the A and the B represent the A-chain and the B-chain respectively. Further, an internal disulphide bridge between Cys(A6) and Cys(A11) may be present or absent.

Compared to human insulin the other insulin receptor ligand is usually different in that it contains one or more mutations in the A-chain and/or one or more mutations in the B-chain. Alternatively or in addition, the ligand has one or more additional amino acid residues at the C-terminal end of the A-chain or B-chain. Alternatively to one or more mutations and/or an elongation at the C-terminal end of the A-chain and/or the B-chain, or in addition to one or more of said differences, one or more amino acid residues of the insulin receptor ligand is derivatised. Groups that can be derivatized are amino groups, carboxylic acid groups and hydroxyl groups. An amino or an hydroxyl group can in particular be derivatised with a carboxylic acid, more in particular a fatty acid having 8-24 carbon atoms, e.g. myristic acid or hexadecanedioic acid. A carboxylic acid group can in particular be derivatized with a compound having an amino group or a compound having a hydroxyl group.

A mutant insulin receptor ligand usually has one, two, three, four or five mutations compared to human insulin. It is in particular preferred that one or more of the mutations are present in the B-chain. Preferred B-chain mutations are: P28K, K29P, P28D, B3K, K29E. Preferred combined mutations are P28K&K29P respectively B3K&K29E. Further insulin receptor ligands are, e.g., those that have been described in EP-A 2 6784 085, EP-A 425 482 or EP-A 419 504, of which the contents, in particular with respect to the insulin receptor ligands in the claims of these documents, are incorporated by reference.

Preferred examples of insulin receptor ligands wherein a side-group is derivatized are ligands wherein K29 of the B-chain contains a fatty acid moiety such as myristic acid, or - hexadecanedioic acid (via a gamma-L-glutamyl spacer).

Specifically preferred insulin receptor ligands other than human insulin are selected from the group of Humalog, aspart, insulin glulisine, insulin detemir, insulin degludec and glargine insulin.

The sequence of subtilisin BPN' is given in SEQUENCE ID NO: 2 (mature form). The gene encoding for subtilisin BPN' amino acids -107 to 275 is given in SEQUENCE ID NO: 1. The subtilisin BPN' variant or homologue can be selected from the enzymes according to WO 2016/056913, with the proviso that it has the above mentioned mutations.

SEQUENCE ID NO: 3 shows subtilisin BPN' variant with deletion of Ca²⁺ binding loop and S221C and the P225 mutation (denoted as P225X). SEQUENCE ID NO: 4 shows a subtilisin BPN' variant wherein the M222X and the Y217X are shown as mutation points. It also shows preferred positions for one or more further mutations. The 'X' may in principle be any amino acid and is usually selected from the mutations mentioned in WO 2016/056913, incorporated herein by reference.

The mutation at the amino acid position corresponding to S221 preferably is S221C.

The mutation at the amino acid position corresponding to P225 is preferably selected from the group of P225N, P225D, P225S, P225C and P225G, more preferably P225N or P225D.

The subtilisin BPN' or homologue thereof preferably comprises one or more mutations selected from the group of mutations at an amino acid position corresponding to Q2, S3, P5, S9, I31, K43, M50, A73, E156, G166, G169, S188, Q206, N212, N218, T254 and Q271 of SEQUENCE ID NO 2. Preferably said one or more mutations are selected from the group Q2K, S3C, P5S, S9A, I31L, K43N, M50F, A73L, E156S, G166S, G169A, S188P, Q206C, N212G, N218S, T254A and Q271E. In a particular preferred embodiment, it comprises at least six, preferably at least eight, more in particular at least 12 of said mutations selected from the group of Q2K, S3C, P5S, S9A, I31L, K43N, M50F, A73L, E156S, G166S, G169A, S188P, Q206C, N212G, N218S, T254A and Q271E.

In a preferred embodiment the subtilisin BPN' variant or homologue thereof comprises one or more mutations at the amino acid position corresponding to N62, G100, S125, L126, G127, P129, N155, and N218 of SEQUENCE ID NO 2.

In a specific embodiment, the subtilisin BPN' variant or homogue thereof comprises comprises mutations at the amino acid positions corresponding to M222 and Y217, wherein the mutations are:
- M222P and Y217H;
- M222P and Y217G;
- M222G and Y217F; or
- M222G and Y217G;

In a further embodiment, the subilisin BPN' or homologue thereof, comprises at least one mutation selected from the group of mutations at an amino acid position corresponding to Y104, I107, L126, S101, G102, G127, G128, L135 and P168 of SEQUENCE ID NO 2, which mutation or mutations may in particular be selected from the group of Y104F, Y104S, I107V, I107A, L135N, L135S, L135D or L135A.

In a method of the invention, the enzymatic coupling reaction is performed in an aqueous fluid Preferably the reaction is performed in a buffered fluid. The inventors realized that a high water content of the reaction medium is advantageous to establish a shielding of the N-terminal glycine of the A-chain. The water content therefore usually is 70-100 vol %, based on total liquids, preferably 80 vol. % or more, more preferably 90 vol. % or more, in particular 95 vol. % or more in particular 98 vol. % or more.

In principle, any buffer is suitable, provided that the buffer does not substantially disrupt the shielding of the N-terminal glycine in the A-chain. Good buffers are known to a person skilled in the art. See for instance David Sheehan in Physical Biochemistry, 2nd Ed. Wiley-VCH Verlag GmbH, Weinheim 2009; http://www.sigmaaldrich.com/life-science/core-bioreagents/biological-buffers/learning-center/buffer-calculator.html.

The pH of the buffer for the coupling reaction may be at least 5, in particular at least 6, preferably at least 7. A desired maximum pH is usually less than 11, in particular less than 10, even more preferably less than 9.

Usually the molar ratio of (a) the (oligo)peptide C-terminal ester or thioester to (b) the insulin receptor ligand is at least about 1:1. The (oligo)peptide C-terminal ester or thioester is preferably in stoichiometiric excess relative to the insuline receptor ligand, in particular in the range of 3:1 to 10:1, more in particular in the range of 5:1 to 10:1.

In a method of the invention, preferably substantial amounts of hydrogen-bond disruptive agents are usually avoided. Examples thereof are disruptive co-solvents, e.g. N,N-dimethylformamide (DMF), N-methylpyrrolidinone (NMP), N,N-dimethylacetamide (DMA), dimethylsulphoxide (DMSO), acetonitrile, tetrahydrofuran (THF), 2-methyl-tetrahydrofuran (Me-THF), 1,2-dimethoxyethane, (halogenated) alcohols; disruptive salts, e.g. guanidinium salts, or disruptive surface active compounds, e.g. sodium dodecyl sulphate or tween. Depending on the stability of the subtilisin BPN' variant or homologue and the insulin receptor ligand, the amount of co-solvent is preferably below 40 vol%, more preferably below 30 vol%, even more preferably below 20 vol%, and most preferably below 10%. Good results have been achieved in a method wherein the aqueous fluid is essentially free of said disruptive co-solvents, said disruptive salts and said disruptive surface active compounds.

In principle the temperature during the enzymatic coupling of the (oligo)peptide (thio)ester with the insulin receptor ligand is not critical, as long as a temperature is chosen at which the subtilisin BPN' variant or homologue used show sufficient activity and stability. Such a temperature is usually known for the subtilisin BPN' variant or homologue to be used or can be routinely determined, making use of a known substrate for the subtilisin BPN' variant or homologue under known reaction conditions. Generally, the temperature may be at least -10 °C, in particular at least 0 °C or at least 10 °C. Generally, the temperature may be 70°C or less, in particular 60°C or less or 50 °C or less. Optimal temperature conditions can easily be identified for a specific subtilisin BPN' variant or homologue for a specific enzymatic coupling reaction by a person skilled in the art through routine experimentation based on common general knowledge and the information disclosed herein. In general, the temperature advantageously is in the range of 20-50°C.

The invention will now be illustrated by the following examples.

### EXAMPLES

### Materials and methods.

The human insulin was used as obtained from Sigma-Aldrich. The production of the subtilisin BPN' mutants and the synthesis of the (oligo)peptides was performed according to WO 2016/056913, see the Examples, page 41-49 of which the contents are incorporated by reference.

### Example 1: Coupling of a pentapeptide selectively to the N-terminus of the A-chain of human insulin.

5 mg of human insulin (Cas # 11061-68-0) and 2.5 mg of Ac-Asp-Phe-Ser-Lys-Leu-OCam-Leu-OH.TFA were dissolved in 200 µL DMF. Subsequently, 200 µL of phosphate buffer (1 M, pH 8.0) and 200 µL H2O containing 20 µg of Subtilisin BPN' containing the following mutations according to SEQ ID 2 (Δ 75-83), Q2K, S3C, P5S, S9A, I31L, K43N, M50F, A73L, E156S, G166S, G169A, S188P, Q206C, N212G, Y217L, N218S, S221C, M222G, P225A, T254A and Q271E was added and the reaction mixture was shaken (150 rpm) at room temperature. After 60 min a 100 µL aliquot of the reaction mixture was withdrawn and quenched with 500 µL MSA/water (1/99, v/v) and analyzed by LC-MS, showing that 92% of the insulin starting material was converted to a single product, i.e. with Ac-Asp-Phe-Ser-Lys-Leu- coupled to the N-terminus of the insulin A-chain.

### Example 2: Coupling of a pentapeptide to the N-terminus of the A- and B-chain of human insulin.

5 mg of human insulin (Cas # 11061-68-0) and 5 mg of Ac-Asp-Phe-Ser-Lys-Leu-OCam-Leu-OH.TFA were dissolved in 200 µL DMF. Subsequently, 200 µL of phosphate buffer (1 M, pH 8.0) and 200 µL H2O containing 55 µg of 20 µg of Subtilisin BPN' containing the following mutations according to SEQ ID 2 (Δ 75-83), Q2K, S3C, P5S, S9A, I31L, K43N, M50F, A73L, E156S, G166S, G169A, S188P, Q206C, N212G, Y217F, N218S, S221C, M222G, P225A, T254A and Q271E was added and the reaction mixture was shaken (150 rpm) at room temperature. After 60 min a 100 µL aliquot of the reaction mixture was withdrawn and quenched with 500 µL MSA/water (1/99, v/v) and analyzed by LC-MS, showing that the insulin starting material was completely consumed and converted to three product peaks, i.e. 1) Ac-Asp-Phe-Ser-Lys-Leu- coupled to the N-terminus of the Insulin A-chain (22 area%), 2) Ac-Asp-Phe-Ser-Lys-Leu- coupled to the N-terminus of the insulin B-chain (3 area%) and 3) Ac-Asp-Phe-Ser-Lys-Leu- coupled to the N-terminus of both the Insulin A- and B-chain (75 area%).

### Example 3: Coupling of a pentapeptide selectively to the N-terminus of the B-chain of human insulin.

5 mg of human insulin (Cas # 11061-68-0) and 5 mg of Ac-Asp-Phe-Ser-Lys-Leu-OCam-Leu-OH.TFA were dissolved in 300 µL water. Subsequently, 100 µL of phosphate buffer (1 M, pH 8.5) and 50 µL H2O containing 5 µg of Subtilisin BPN' containing the following mutations according to SEQ ID 2 (Δ 75-83), Q2K, S3C, P5S, S9A, I31L, K43N, M50F, A73L, E156S, G166S, G169A, S188P, Q206C, N212G, Y217F, N218S, S221C, M222G, P225N, T254A and Q271E was added and the reaction mixture was shaken (150 rpm) at room temperature. After 60 min a 100 µL aliquot of the reaction mixture was withdrawn and quenched with 500 µL MSA/water (1/99, v/v) and analyzed by LC-MS, showing that 72% of the insulin starting material was converted to two products, i.e. Ac-Asp-Phe-Ser-Lys-Leu-coupled to the N-terminus of the insulin B-chain (95% area%) and Ac-Asp-Phe-Ser-Lys-Leu-coupled to the N-terminus of the insulin A-chain (5% area%).

### SEQUENCES

SEQ ID NO 1: wild type gene encoding for subtilisin BPN' amino acids -107 to 275 ENA |K02496| K02496.1 B. Subtilisin BPN' Bacillus amyloliquefaciens 
GTGAGAGGCAAAAAAGTATGGATCAGTTTGCTGTTTGCTTTAGCGTTAATCTT
TACGATGG
CGTTCGGCAGCACATCCTCTGCCCAGGCGGCAGGGAAATCAAACGGGAAAA
GAAATATAT
TGTCGGGTTTAAACAGACAATGAGCACGATGAGCGCCGCTAAGAAGAAAGAT
GTCATTTCT
GAAAAAGGCGGGAAAGTGCAAAAGCAATTCAAATATGTAGACGCAGCTTCAGC
TACATTAA
ACGAAAAAGCTGTAAAAGAATTGAAAAAAGACCCGAGCGTCGCTTACGTTGAA
GAAGATCA
CGTAGCACATGCGTACGCGCAGTCCGTGCCTTACGGCGTATCACAAATTAAAG
CCCCTGCT
CTGCACTCTCAAGGCTACACTGGATCAAATGTTAAAGTAGCGGTTATCGACAG
CGGTATCG
ATTCTTCTCATCCTGATTTAAAGGTAGCAGGCGGAGCCAGCATGGTTCCTTCT
GAAACAAA
TCCTTTCCAAGACAACAACTCTCACGGAACTCACGTTGCCGGCACAGTTGCGG
CTCTTAAT
AACTCAATCGGTGTATTAGGCGTTGCGCCAAGCGCATCACTTTACGCTGTAAA
AGTTCTCG
GTGCTGACGGTTCCGGCCAATACAGCTGGATCATTAACGGAATCGAGTGGGC
GATCGCAAA
CAATATGGACGTTATTAACATGAGCCTCGGCGGACCTTCTGGTTCTGCTGCTT
TAAAAGCG
GCAGTTGATAAAGCCGTTGCATCCGGCGTCGTAGTCGTTGCGGCAGCCGGTAA
CGAAGGCA
CTTCCGGCAGCTCAAGCACAGTGGGCTACCCTGGTAAATACCCTTCTGTCATT
GCAGTAGG
 
CGCTGTTGACAGCAGCAACCAAAGAGCATCTTTCTCAAGCGTAGGACCTGAGC
TTGATGTC
ATGGCACCTGGCGTATCTATCCAAAGCACGCTTCCTGGAAACAAATACGGGGC
GTACAACG
GTACGTCAATGGCATCTCCGCACGTTGCCGGAGCGGCTGCTTTGATTCTTTCT
AAGCACCC
GAACTGGACAAACACTCAAGTCCGCAGCAGTTTAGAAAACACCACTACAAAAC
TTGGTGAT
TCTTTCTACTATGGAAAAGGGCTGATCAACGTACAGGCGGCAGCTCAGTAA
SEQ ID NO 2: wild type subtilisin BPN' (mature)
   >SUBT_BACAM Subtilisin BPN' Bacillus amyloliquefaciens mature 1 to 275
SEQ ID NO 3: subtilisin BPN' variant with deletion of Ca²⁺ binding loop and S221C and preferably P225 mutation (denoted as P225X) 
AQSVPYGVSQIKAPALHSQGYTGSNVKVAVIDSGIDSSHPDLKVAGGASMVPSE
TNPFQDNNSHGTHVAGTVAAVAPSASLYAVKVLGADGSGQYSWIINGIEWAIAN
NMDVINMSLGGPSGSAALKAAVDKAVASGVVVVAAAGNEGTSGSSSTVGYPGK
YPSVIAVGAVDSSNQRASFSSVGPELDVMAPGVSIQSTLPGNKYGAYNGTCMAS
XHVAGAAALILSKHPNWTNTQVRSSLENTTTKLGDSFYYGKGLINVQAAAQ
SEQ ID NO 4: subtilisin BPN' variant with preferred mutation positions compared to SEQ ID NO 3 
AXXVXYGVXQIKAPALHSQGYTGSNVKVAVXDSGIDSSHPDLXVAGGASXVPSE
TNPFQDNNSHGTHVAGTVXAVAPSASLYAVKVLGADGSGQYSWIINGIEWAIAN
NMDVINMSLGGPSGSAALKAAVDKAVASGVVVVAAAGNXGTSGSSSTVXYPXK
YPSVIAVGAVDSSNQRAXFSSVGPELDVMAPGVSIXSTLPGXKYGAXXGTCXASX
HVAGAAALILSKHPNWTNTQVRSSLENTXTKLGDSFYYGKGLINVXAAAQ
SEQ ID NO 5: The A-chain of human insulin:
   GIVEQCCTSI CSLYQLENYC N
SEQ ID NO 6: The B-chain of human insulin:
   FVNQHLCGSH LVEALYLVCG ERGFFYTPKT

## Claims

1. A method for enzymatically coupling an (oligo)peptide C-terminal ester or thioester to an insulin or other insulin receptor ligand having a first peptide chain comprising an N-terminal glycine residue (also known as an A-chain) and a second peptide chain having an N-terminal phenylalanine residue (also known as a B-chain), wherein the C-terminal ester or thioester is coupled at a higher rate to the N-terminal phenylalanine residue of the second peptide chain than to the N-terminal glycine residue of the first peptide chain,
which coupling is carried out in an aqueous fluid wherein the N-terminal glycine residue is at least substantially shielded by a remaining part of the insulin or other insulin receptor ligand, and
which coupling is catalysed by a subtilisin BPN' variant or a homologue thereof, which comprises the following mutations compared to subtilisin BPN' represented by SEQUENCE ID NO: 2 or a homologue sequence thereof:
- a deletion of the amino acids corresponding to positions 75-83;
- a mutation at the amino acid position corresponding to S221, the mutation being S221C or S221selenocysteine;
- a mutation at the amino acid position corresponding to P225 selected from the group of P225N, P225D, P225S, P225C, P225G, P225A, P225T, P225V, P225I an P225L;
- a mutation at the amino acid position corresponding to M222 selected from the group M222P and M222G;
- a mutation at the amino acid position corresponding to Y217 selected from the group of Y217F, Y217H, Y217G, Y217L, Y217A, Y217N, Y217E,, Y217R, Y217K and Y217P;
wherein the amino acid positions are defined according to the sequence of subtilisin BPN' represented by SEQUENCE ID NO: 2.

2. Method according to claim 1, wherein the ratio of the coupling towards said N-terminal phenylalanine to said N-terminal glycine is at least 3:1, in particular 6:1 to 100:1, more in particular 10:1 to 100:1.

3. Method according to claim 1 or 2, wherein the aqueous fluid comprises 80-100 vol% water, preferably 90-100 vol% water, based on total solvents.

4. Method according to any of the preceding claims, wherein the aqueous fluid is free of hydrogen-bond disruptive agents in an effective amount to disrupt hydrogen bonds between amino acid residues of one or both of said peptide chains, such as disruptive co-solvents, e.g. N,N-dimethylformamide (DMF), N-methylpyrrolidinone (NMP), N,N-dimethylacetamide (DMA), dimethylsulphoxide (DMSO), acetonitrile, tetrahydrofuran (THF), 2-methyl-tetrahydrofuran (Me-THF), 1,2-dimethoxyethane, (halogenated) alcohols; disruptive salts, e.g. guanidinium salts, or disruptive surface active compounds, e.g. sodium dodecyl sulphate.

5. Method according to any of the preceding claims, wherein the subtilisin BPN' variant or homologue thereof has a mutation at the amino acid position corresponding to P225 selected from the group of P225N, P225D, P225S, P225C or P225G.

6. Method according to any of the preceding claims, wherein the subtilisin BPN' variant or homologue thereof has a mutation at the amino acid position corresponding to a mutation at the amino acid position corresponding to Y217 selected from the group of Y217F, Y217H, Y217G, Y217L, Y217A, Y217N, Y217E,, Y217R , Y217K and Y217P, in particular Y217F, Y217H and Y217G.

7. Method according to any of the preceding claims, wherein the subtilisin BPN' variant or homologue thereof, comprises at least four, preferably at least six, more preferably at least eight, more preferably at least 12 of said mutations selected from the group of Q2K, S3C, P5S, S9A, I31L, K43N, M50F, A73L, E156S, G166S, G169A, S188P, Q206C, N212G, N218S, T254A and Q271E.

8. Method according to any of the preceding claims, wherein the insulin or other insulin receptor ligand is human insulin or a insulin receptor ligand comprising said first peptide chain and said second peptide chain, with in total 5 or less mutations in the amino acid sequences of the first chain plus the second chain, compared to the corresponding first and second chain of human insulin, with the proviso that the insulin receptor ligand comprising a first chain having an N-terminal glycine and a second chain having an N-terminal phenylalanine, and wherein the first and second chain are bound by disulphide bridges.

9. Method according to any of the preceding claims, wherein the insulin receptor ligand comprises a non-peptidic moiety at a side-chain functionality of one or more amino acid residues in the A- and/or B-chain, in particular a fatty acid residue, which may be bound directly to the side-chain functionality or bound via a spacer.

10. Method according to any of the preceding claims, wherein the insulin receptor ligand is selected from the group of human insulin, porcine insulin, Humalog, aspart, insulin glulisine, insulin detemir, insulin degludec, glargine insulin.

11. Method according to any of the preceding claims, wherein the (oligo)peptide of the (oligo)peptide C-terminal ester or thioester is selected from the group of immunoglobulins, globular proteins, or pharmaceutically active peptides.

12. Method according to any of the claims 1-10, wherein the (oligo)peptide of the (oligo)peptide C-terminal ester or thioester is a spacer peptide of which the N-terminal end and/or one or more side-chain functionalities is/are coupled to a non-peptide moiety altering an ADME characteristic of the insulin receptor ligand.

13. Method according to any of the preceding claims, wherein the (oligo)peptide of the (oligo)peptide C-terminal ester or thioester is a conjugate of an (oligo)peptide C-terminal ester or thioester and a moiety selected from the group of polyalkylene glycols (in particular polyalkylene glycol) fatty acids, and polysialic acids.

14. Method according to any of the preceding claims, wherein the (oligo)peptide ester or thioester is used in a stoichiometric excess, preferably in a molar ratio of oligo(peptide) ester or thioester to insulin receptor ligand of at least 3:1, in particular of 5:1 to 10:1.

15. Method according to any of the preceding claims, wherein a coupling product of (oligo)peptide ester or thioester and insulin receptor ligand is obtained comprising a conjugate having a single (oligo)peptide coupled to the insulin receptor ligand, the single (oligo)peptide being coupled the N-terminal phenylalanine of the B-chain, in a relative amount of at least 80 mol %, based on total coupling product, preferably of at least 90 mol %, based on total coupling product, without needing any separation or other recovery step.
